# EUROPÄISCHE PATENTSCHRIFT

(11) **EP 2 437 896 B1**
(45) Veröffentlichungstag und Bekanntmachung des Hinweises auf die Patenterteilung: **02.09.2015**
(21) Anmeldenummer: 10724069.9
(22) Anmeldetag: 02.06.2010
(51) Int. Cl.: B05B 17/06, A61M 11/00, A61M 15/00, B23K 11/00, B23K 11/22, B23K 11/093

(54) **MEMBRANVERNEBLER UND VERFAHREN ZUM VERSCHWEIßEN EINER MEMBRAN MIT EINEM TRÄGER BEI DER HERSTELLUNG EINES MEMBRANVERNEBLERS**
MEMBRANE NEBULIZER AND METHOD FOR WELDING A MEMBRANE TO A CARRIER DURING THE PRODUCTION OF A MEMBRANE NEBULIZER
NÉBULISEUR À MEMBRANE ET PROCÉDÉ DE SOUDAGE D'UNE MEMBRANE À UN SUPPORT LORS DE LA FABRICATION D'UN NÉBULISEUR À MEMBRANE

(30) Priorität: 02.06.2009 DE 102009026636
(43) Veröffentlichungstag der Anmeldung: 11.04.2012
(73) Patentinhaber: PARI Pharma GmbH, 82319 Starnberg (DE)
(72) Erfinder: PUMM, Gerhard, 82496 Oberau (DE); SEIFERT, René, 82418 Murnau (DE); HOLZMANN, Philipp, 81371 München (DE); BRUNE, Nicole, 82110 Germering (DE)
(74) Vertreter: Hoffmann Eitle
(86) Internationale Anmeldenummer: PCT/EP2010/057718
(87) Internationale Veröffentlichungsnummer: WO 2010/139730

(56) Entgegenhaltungen:
- WO-A2-01/76762
- WO-A2-2006/127181
- US-A- 4 420 848
- US-B1- 6 689 981

## Beschreibung

Die vorliegende Erfindung betrifft einen Membranvernebler zur Erzeugung von Flüssigkeitströpfchen mit einer in Schwingungen versetzten Membran, insbesondere für die Verneblung von Fluiden, insbesondere Flüssigkeiten für therapeutische Zwecke, Mit anderen Worten betrifft die vorliegende Erfindung einen Membranvernebler zur Aerosolerzeugung in einer Aerosoltherapievorrichtung. Ferner betrifft die vorliegende Erfindung auch ein Verfahren zum Verbinden einer Membran mit einem flächigen Träger bei der Herstellung eines solchen Membranverneblers.

Ein Membranvernebler mit den Merkmalen des Oberbegriffs von Anspruch 1 ist beispielsweise aus der DE 101 22 065 A1 oder der DE 10 2005 006 375 A1 bekannt. Ein Membranvernebler mit den Merkmalen im Oberbegriff von Anspruch 1 geht aus der WO-A-01/176762 hervor. Eine ähnliche Offenbarung findet sich auch in der WO-A-2006/127 189. Die US-A-4,420,848 offenbart ein Widerstandsschweißverfahren zum Verbinden von Kappen für Radmuttern, während die US-A-6,689,981 ein Widerstandsschweißverfahren zum Verschließen eines Rohres beschreibt.

Bei der Herstellung eines solchen Membranverneblers muss die Membran mit dem Aktuator, der die Membran in Schwingungen versetzt, insbesondere einem Piezoschwinger, verbunden werden. Zurzeit erfolgt diese Verbindung über eine Klebung der Membran auf den Träger unter Verwendung eines Klebemittels. Nachteilig daran ist zum einen die lange Prozessdauer, die vor allem auf die Aushärtezeit des Klebemittels zurückzuführen ist. Des Weiteren ist die Verwendung von Klebemitteln grundsätzlich mit einer schwierigen Handhabung, wie spezielle Presstools, in einem kontinuierlichen Fertigungsprozess verbunden. Des Weiteren ist es problematisch ein geeignetes Klebemittel zu finden, da dieses medizinisch zugelassen und mehrfach autoklavierbar (derzeitige Mindestanforderung 50 Zyklen) sein muss. Darüber hinaus müssen der Träger und die Membran im vorfeld behandelt, z. B, sandgestrahlt und gereinigt werden, um die Haftung über das Klebemittel zu verbessern.

Der vorliegenden Erfindung liegt daher die Aufgabe zu Grunde einen Membranvernebler zu schaffen, der bei gleichem Schwingungsverhalten der Membran und somit gleicher Aerosolerzeugung einfacher, schneller und damit kostengünstiger hergestellt werden kann sowie ein Verfahren zum Verbinden einer Membran mit einem Träger bei der Herstellung eines Membranverneblers vorzuschlagen, das einfacher, schneller und kostengünstiger als das bisherige Klebeverfahren ist.

Diese Aufgabe wird durch einen Membranvernebler mit den Merkmalen des Anspruchs 1 und ein Verfahren mit den Merkmalen des Anspruchs 5 gelöst. Vorteilhafte Weiterbildungen der vorliegenden Erfindung finden sich in den Unteransprüchen.

Als problematisch stellt es sich bei der Lösung dieser Aufgabe dar, dass die Verbindungsart zwischen Membran und Träger (bzw. mit dem Aktuator, insbesondere dem Piezoschwinger) einen erheblichen Einfluss auf das Schwingungsverhalten und somit die Aerosolerzeugung der Membran hat. Alle metallischen Bauteile inklusive dem Aktuator bilden zusammen einen Biegeschwinger mit einem charakteristischen Schwingverhalten. Dies beinhaltet typische Amplituden, Resonanzen und Leistungsumsetzung, welche je nach Abstimmung der Baugruppen und Herstellung realisiert werden. Darüber hinaus muss im Bereich der Verbindung der Membran mit dem Träger eine dichte und vollständig geschlossene, z.B. ringförmige, Verbindung hergestellt werden, da es andernfalls zu unkontrolliertem Fluid, insbesondere Medikamentenaustritt, bei der Verneblung kommen kann. Schließlich handelt es sich insbesondere bei der Membran aber auch bei dem Träger um sehr dünne, metallische Bauteile mit einer Stärke im Bereich von 25 um bzw. 500 um (die Wandstärke der Membran liegt in einem Bereich zwischen 25 µm und 200 µm und die des Trägers in einem Bereich zwischen 50 µm und 500 µm). Damit scheiden im Prinzip Verbindungsverfahren mit einer hohen Wärmeentwicklung aus, weil durch die Wärmeentwicklung mit einem Verzug oder Undichtigkeit (Leckage) der beiden zu verbindenden Bauteile zu rechnen ist und damit eine erhebliche Beeinflussung des Schwingungsverhaltens und der Aerosolerzeugung.

Überraschenderweise hat sich jedoch herausgestellt, dass ein Verschweißen der Membran mit dem Träger unter Verwendung eines Widerstandsschweißverfahren mit einem so geringen Verzug der Bauteile möglich ist, dass Schwingungsverhalten und Aerosolerzeugung nicht beeinflusst zumindest aber handhabbar bleiben. Dementsprechend liegt der vorliegenden Erfindung der Gedanke zu Grunde die Membran und den flächigen Träger eines Membranverneblers miteinander zu verschweißen, wobei insbesondere ein Widerstandsscheißverfahren zum Einsatz kommt.

Gemäß der vorliegenden Erfindung umfasst ein Membranvernebler zur Aerosolerzeugung in einer Aerosoltherapievorrichtung bzw. zur Erzeugung von Flüssigkeitströpfchen eine Membran mit mehreren Durchgangslöchern zur Verneblung eines Fluids. Hierzu ist ein Piezoschwinger vorgesehen, der die Membran in Schwingungen versetzt und damit das Fluid durch die Durchgangslöcher hindurch von einer Seite zur anderen vernebelt wird. Bezüglich dieser Funktionsweise wird der Fachmann hinsichtlich weiterer Details auf die DE 101 22 065 A1 verwiesen. Darüber hinaus ist ein flächiger Träger vorgesehen, der eine (vorzugsweise kreisrunde) Öffnung aufweist, wobei die Membran in der Öffnung angeordnet ist bzw. über der Öffnung angeordnet ist und am Träger befestigt ist, so dass auf einer Seite der Membran das Fluid anstehen kann, während es auf der entgegengesetzten Seite der Membran vernebelt wird. Hierzu ist die Membran derart in der Öffnung angeordnet an dem Träger befestigt, dass auf einer ersten Seite des Trägers die Vernebelung stattfindet und auf der entgegengesetzten zweiten Seite des Trägers das Fluid an der Membran ansteht. Die vorliegende Erfindung kennzeichnet sich dadurch, dass die Membran vollumfänglich mit dem Träger verschweißt ist. Hierbei kommt ein Widerstandsschweißverfahren zum Einsatz, Es können hierbei zum Beispiel Mittelfrequenzschweißverfahren und Kondensatorentladungsschweißverfahren zur Anwendung kommen. Beim Widerstandsschweißen werden reproduzierbare Schweißergebnisse bei einer gleichzeitigen Minimierung der Produktionskosten und damit eine Erhöhung der Wirtschaftlichkeit erreicht. Weitere Vorteile sind minimierte Anschlusskosten dank geringer Anschlusswerte sowie niedrige Energiekosten durch optimalen Leistungsfaktor.

Beim Mittelfrequenzschweißverfahren wird mithilfe von modernen Inverter-Stromquellen die benötigte Energie für die Schweißprozesse mit einem nahezu beliebigen Impulsverlauf geregelt zugeführt. Die Anschluss-Netzspannung wird im Inverter zunächst gleichgerichtet und dann über einen geregelten Wechselrichter sowie einen Transformator als getaktete Gleichspannung bereitgestellt. Mittelfrequenzschweißverfahren wird verwendet bei unterschiedlichen Materialien wie Aluminium, VA und beschichteten Blechen, Dabei ist auch die Verbindung von Materialien unterschiedlicher Wärmeleitfähigkeit, wie Aluminium auf Stahl möglich.

Beim Kondensatorentladungsschweißen wird die zum Schweißen benötigte Energie aus einer vorher geladenen Kondensatorenbank auf eine oder mehrere Schweißelektroden geschaltet. Durch die schnelle Entladung der in den Kondensatoren gespeicherten Energie steigt der Strom im Sekundärkreis sehr schnell an, wodurch auch die Temperatur der Schweißstelle ebenso schnell ansteigen kann. Dieser schnelle Temperaturanstieg erhitzt die Schweißzone bevor die Wärme abfließen kann und verhindert somit eine Erwärmung der Bereiche um die Schweißstelle bzw. den Schweißbereich. Dadurch kann eine kurze Schweißzeit bei geringem Energiebedarf erreicht werden. Durch den geringen Wärmeeintrag wird ferner ein stabiler Prozess und ein genauer Schweißbereich erzielt sowie der Verzug der sehr dünnen Bauteile in einem akzeptablen Maß bzw. in einem handhabbaren Maß gehalten.

Durch dieses Verbindungsverfahren wird die Möglichkeit geschaffen die Membran flächig auf der ersten Seite des Trägers, d. h. auf der Seite des Trägers, auf der die Verneblung stattfindet aufzubringen. Dies hat im Vergleich zu einem Auflegen auf der zweiten Seite den Vorteil, dass das Schwingungsverhalten des gesamten Bauteils (Biegeschwinger) in dem gewünschten Anwendungsfall positiv beeinflusst wird. Zusätzlich ist eine Fertigung des Bauteils von einer Seite möglich. Erzielt wird somit eine flüssigkeitsundurchlässige (dichte) Verbindungsform, welche sich mechanisch und elektrisch stabil verhält und sich in gewissen Toleranzgrenzen reproduzierbar fertigen lässt.

Als vorteilhaft hat sich beim Kondensatorschweißen gegenüber dem Mittelfrequenzschweißen die geringeren notwendigen elektrischen Versorgungsleistungen erwiesen. Beim Mittelfrequenzschweißen wird ein Strom von ca. 300 - 500 A benötigt, beim Kondensatorentladungsschweißen reichen dagegen Ströme bis zu 64 A. Generell sind beide Verfahren für diese Anwendung geeignet.

Der Vorteil dieser Widerstandsschweißverfahren ist die direkte Qualitätskontrolle anhand der gemessenen widerstände und Ströme. Dies ermöglicht eine In-Prozess Kontrolle und erspart weitere Qualitätskontrollmessungen. Zum Beispiel kann beim alternativen Laserschweißen die Dichtigkeit nicht direkt gemessen werden und es wäre zum Beispiel eine separate Kamerakontrollmessung nötig.

Dabei umfasst die Membran einen effektiven Bereich, der z. B. kreisförmig sein kann und in dem die Durchgangslöcher angeordnet sind sowie einen den effektiven Bereich vollumfänglich umgebenden, vorzugsweise ringförmigen, Befestigungsbereich zur Befestigung an dem Träger. Der Befestigungsbereich ist als auf dem Träger flächig aufliegender Kragen ausgestaltet. Wie es zuvor erwähnt wurde, liegt der Kragen auf der ersten Seite des Trägers flächig auf und der effektive Bereich ist vorzugsweise zur Öffnung des Trägers zentriert ausgerichtet ist. Hierbei kann die Öffnung beispielsweise gleichfalls kreisförmig sein und der effektive Bereich konzentrisch zur Öffnung angeordnet sein.

Dabei kann durch die vorliegende Erfindung der flächig aufliegende Kragen im Vergleich zu einer Klebemittelverbindung und aufgrund der Stärke (Festigkeit) der Schweißverbindung und der erzielbaren Dichtigkeit mit einer sehr geringen Fläche ausgestaltet werden, die vorzugsweise weniger als 96 mm², bevorzugt weniger als 80 mm² , bevorzugter weniger als 40 mm² und am meisten bevorzugt weniger als 20 mm² beträgt. Bevorzugterweise ist die Fläche jedoch ≥ 5 mm² und am meisten bevorzugt größer gleich 10 mm² . Dadurch wird einerseits der Materialeinsatz reduziert, die Schweißnaht optimiert und andererseits kann auf vorteilhafte Weise das gewünschte Schwingungsverhalten der Bauteile (bzw. des Biegeschwingers) erzielt werden.

Wie es bereits eingangs erwähnt wurde, können die Membran und/oder der Träger aus Edelstahl oder einem anderen metallischen Werkstoff, der zur medizinischen Verwendung geeignet und zugelassen ist, gebildet sein. Dabei liegt die Wandstärke der Membran vorzugsweise unter 200 µm, bevorzuge zwischen 25 µm und 200 µm, und am meisten bevorzugt zwischen 50 µm und 120 µm. Die Wandstärke des Trägers liegt vorzugsweise unter 500 µm, bevorzugt zwischen 50 µm und 500 µm, am meisten bevorzuge zwischen 100 µm und 400 µm.

Darüber hinaus ist, wie erwähnt, ein Piezaschwinger vorgesehen, um zumindest die Membran zur Vernebelung des Fluids in Schwingungen zu versetzen, wobei der Piezaschwinger mit dem Träger verbunden, z. B. verklebt sein kann. Er ist auf der gleichen Seite wie die Membran angeordnet. Dabei liegt die Wandstärke des Piezaschwingers in vergleichbaren Größenverhältnissen und vorzugsweise unter 500 µm, bevorzugt zwischen 25 µm und 500 µm und am meisten bevorzugt zwischen 100 µm und 400 µm.

Neben dem oben erwähnten Membranvernebler schlägt die vorliegende Erfindung eine Aerosoltherapievorrichtung mit einem solchen Membranvernebler vor.

Ferner betrifft die vorliegende Erfindung ein Verfahren zum Verbinden einer Membran, insbesondere einer Membran, wie sie oben erläutert wurde, mit einem ebenfalls oben beschriebenen flächigen Träger bei der Herstellung eines Membranverneblers einer Aerosoltherapiervorrichtung. Dabei umfasst das Verfahren die Schritte flächiges Auflegen des Bereichs (Befestigungsbereichs) der Membran (z. B. des Kragens), der mit dem Träger zu verschweißen ist. Hierbei wird der effektive Bereich der Membran zu der Öffnung im Träger entsprechend ausgerichtet, so dass der effektive Bereich über bzw. in der Öffnung liegt. Bei einem kreisförmigen effektiven Bereich und kreisförmiger Öffnung sind die beiden Kreise beispielsweise konzentrisch zueinander. Gleiches gilt auch für den dann z. B. ringförmigen Kragen. Im Anschluss wird eine im Querschnitt geschlossene, z. B. ringförmige Schweißelektrode in dem Bereich (Befestigungsbereich), in dem Träger und Membran zu verschweißen sind, flächig angepresst und zum Verbinden von Membran und Träger ein Widerstandsschweißvorgang (wie, Mittelfrequenzschweißvorgang oder Kondensatorentladungsschweißvörgang) durchgeführt. Dabei hat die z. B. ringförmige Schweißelektrode einen definierten Innen- und Außendurchmesser, der die radiale Stärke (Dicke) der gleichermaßen ringförmigen Schweißnaht definiert.

Um beim Schweißvorgang Korrosionsschäden zu vermeiden, die vor allem durch den anschließenden Kontakt mit unterschiedlichen Lösungen und Reinigungsschritten auftreten können, erfolgt der Schweißvorgang unter Schutzgasatmosphäre, z. B. unter Formiergasen, vorzugsweise Argon, angepasst an die jeweils verwendeten Materialien.

Darüber hinaus kann nicht garantiert werden, dass der Stromfluss über die Schweißelektrode gleichmäßig durch die Bauteile fließt, wodurch eventuell Schweißfehler und Undichtigkeiten auftreten können. Hierbei hat sich herausgestellt, dass die in der Praxis gängigen Buckelschweißverfahren, die üblicherweise ein gleichförmiges Schweißbild erzeugen, bei den vorhandenen Materialstärken nicht optimal anwendbar sind. Die Herstellung eines sauberen. Grates (Buckel) ist in dieser Anwendung nicht reproduzierbar. Um diese Problematik zu vermeiden, wird der Schweißstrom partiell eingeleitet und dieser Vorgang nach Bedarf oder Anwendung ein oder mehrmals wiederholt. Dafür erfolgt ein Verdrehen des Verbunds aus Membran und Träger und der Schweißelektrode relativ zueinander nachdem der erste Widerstandsschweißvorgang (wie Mittelfrequenz- oder Kondensatorentladungsschweißvorgang) durchgeführt wurde, d. h. der Verbund und die Schweißelektrode werden relativ zueinander versetzt, wozu die Relativdrehung weniger als 360° betragen sollte. Im Anschluss wird ein erneuter Schweißvorgang durchgeführt. Dieses Versetzen kann mehrfach, vorzugsweise dreifach, durchgeführt werden, wobei die Relativdrehung jeweils 120° beträgt. Es sind jedoch auch mehrere oder nur zwei Schweißvorgänge denkbar, wobei die Drehung vorzugsweise in gleichmäßigen Teilungen z. B. 180° bei einer zweifachen Schweißung oder 90° bei einer vierfachen Schweißung erfolgt.

Wie es oben erwähnt wurde, ist es aus besagten Gründen vorteilhaft die Membran in dem Bereich, in dem Membran und Träger zu verbinden sind, flächig auf der ersten Seite des Trägers aufzulegen, d. h. auf der Seite des Trägers, auf der die Vernebelung (bzw. Aerosolgenerierung) stattfindet.

Weitere Vorteile und Merkmale, die alleinstehend oder in Kombination mit einem oder mehreren der obigen Merkmale umgesetzt werden können, sind darüber hinaus aus der folgenden Beschreibung einer bevorzugten beispielhaften Ausführungsform ersichtlich, die unter Bezugnahme auf die begleitende Zeichnung erfolgt.

Dabei zeigt:

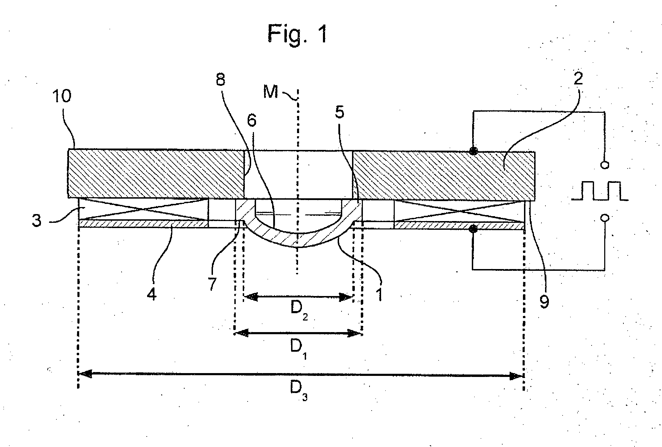

Figur 1 einen Membranvernebler der vorliegenden Erfindung im Querschnitt.

Figur 1 zeigt einen Membranvernebler der vorliegenden Erfindung. Das in Figur 1 dargestellte schwingungsfähige System ist rotationssymmetrisch bezüglich der in Figur 1 dargestellten Mittelachse M. Es umfasst einen gewölbte Membran 1, einen Träger bzw. ein Substrat 2 mit einer zentral angeordneten kreisrunden Öffnung 8. Die gewölbte Membran ist in der Öffnung 8 angeordnet. Die Membran 1 ist gleichfalls kreisrund und konzentrisch zur Mittelachse M angeordnet. Die Membran 1 umfasst einen kreisförmigen, zentral angeordneten, effektiven Bereich, der eine Vielzahl von nicht sichtbaren Durchgangslöchern im Größenbereich unter 10 µm und vorzugsweise zwischen 1,5 µm und 5 µm Durchmesser aufweist. Konzentrisch zum effektiven Bereich 6 ist ein kreisringförmiger Kragen 7 angeordnet, der über die Öffnung 8 hinausragt und der Befestigung der Membran an dem Träger 2 dient. Der Träger 2 weist eine erste Seite 9 und eine entgegengesetzte bzw. gegenüberliegend angeordnete Seite 10 auf. Im verbauten Zustand des Membranverneblers in einer Aerosoltherapievorrichtung steht das zu vernebelnde Fluid auf der Seite 10 und damit über die Öffnung 8 am effektiven Bereich 6 der Membran 1 an. Auf der gegenüberliegenden Seite 9 erfolgt die Vernebelung (bzw. Aerosolerzeugung), wenn das dargestellte System in Schwingung versetzt wird und das Fluid, insbesondere eine Flüssigkeit, durch die mehreren Durchgangslöcher hindurch auf der Seite 9 vernebelt wird (bzw. als Aerosol austritt). Der Träger 2 ist vorzugsweise ebenfalls kreisrund und weist einen Durchmesser D3 von weniger als 30 mm, vorzugsweise weniger als 27 mm auf und besonders bevorzug weniger als 24 mm auf. Darüber hinaus ist auf der gleichen Seite 9 ein Piezoelement 3 an dem Träger 2 befestigt, insbesondere verklebt, und über eine erste Elektrode 4 und über den Träger 2 kann eine Wechselspannung angelegt werden. Dabei kann der Träger 2 die Funktion einer zweiten Elektrode für das Piezoelement 3 übernehmen. Es kann aber auch eine zweite Elektrode auf der Seite 10 des Trägers vorgesehen werden.

Eine an die Elektroden angelegte Wechselspannung führt zu einer Verlängerung bzw. Verkürzung des Piezoelements 3 in einer Richtung senkrecht zu der in Figur 1 gezeigten Symmetrieachse M. Dadurch wird der Träger im Wechsel der Verlängerung und Verkürzung des Piezoelements 3 verbogen und zu Biegeschwingungen angeregt, die auf die Membran 1 übertragen werden. Die Resonanzfrequenzen des Schwingungssystems werden einerseits durch die Membran 1, das Substrat 2 und das Piezoelement 3 sowie die Befestigungsart der Membran 1 am Substrat 2 bestimmt. Andererseits werden die Resonanzfrequenzen des Schwingungssystems zusätzlich beeinflusst durch die Flüssigkeit, die der konkaven Seite der Membran 1 zugeführt wird und dort während des Vernebelns ansteht. Dies gilt im besonderen Maße für therapeutische Inhalationsgeräte (z.B. Medikamentenvernebler), bei denen die Flüssigkeit in einem hierfür vorgesehenen Reservoir in ausreichender Menge unmittelbar an der Membran zur Verfügung gestellt wird.

Die Befestigung der Membran 1 an dem Träger 2 erfolgt hier durch eine Schweißnaht 5 im Bereich des Kragens 7. Hierzu liegt der Kragen 7 flächig auf der Seite 9 des Trägers 2 auf. Die Verbindung erfolgt dabei derart, dass die Membran 1 mit dem Kragen 7 flächig auf den Träger bzw. dessen Seite 9 aufgelegt wird, und im Anschluss eine ringförmige Schweißelektrode (nicht dargestellt) auf die in Figur 1 nach unten weisende Oberfläche des Kragens 7 aufgepresst wird. Nachfolgend entsteht die Verbindung durch einen Widerstandsschweißvorgang, vorzugsweise ein Kondensatorentladungsschweißvorgang. Um eine ausreichend dichte Verbindung, d. h. eine vollständig geschlossene Schweißnaht 5 (ringförmige Schweißnaht) zwischen Membran 1 und dem Träger 2 zu erzielen, wird nachfolgend gemäß einer bevorzugten Ausführungsform der Verbund aus Membran 1 und Träger 2 relativ zur Schweißelektrode oder die Schweißelektrode zum Verbund um 120° gedreht und der Schweißvorgang erneut durchgeführt. Anschließend erfolgt eine weitere Drehung um 120° und ein nochmaliger Schweißvorgang. Es versteht sich jedoch, dass auch nur zwei Schweißvorgänge oder mehr als drei Schweißvorgänge durchgeführt werden können.

Darüber hinaus ist die ringförmige Schweißelektrode in ihrem Innen- und Außenmesser definiert, um die Breite der Schweißnaht in Radialrichtung des Systems einstellen zu können. Darüber hinaus ist auch die Breite des Kragens 7 in Radialrichtung entsprechend angepasst. Die Fläche des Kragens liegt dabei vorzugsweise in einem Bereich zwischen 5 mm² und höchsten 96 mm², vorzugsweise höchstens 80 mm², mehr bevorzugter höchstens 40 mm² und am meisten bevorzugt höchstens 20 mm². Die Fläche wird dabei bemessen in dem Bereich, der über die Öffnung 8 hinausragt, d. h. der Bereich, der zwischen den Durchmessern D2 und D1 in Figur 1 liegt.

Um Korrosion zu vermeiden, wird der oben genannte Schweißvorgang unter Schutzgasatmosphäre z. B. in einer besonderen Atmosphäre mit Formiergasen bzw. vorzugsweise Argon durchgeführt, je nachdem weiche Materialien verschweißt werden.

Durch die vorliegende Erfindung kann die Dauer des Verbindungsvorgangs deutlich reduziert werden, ist unabhängig von Drittmaterial, nämlich Klebstoff, und benötigt keine Vorbehandlung der Materialien, Des Weiteren hat diese Verbindung eine höhere Festigkeit und somit eine höhere Autoklavierbeständigkeit. Ferner kann die Verwendung von Werkstückträgern zur Fixierung der Bauteile im Klebeprozess entfallen, wodurch die Investitionskosten bei Erhöhung der Stückzahlen reduziert werden können.

Es versteht sich, dass die obige Ausführungsform nur eine beispielhafte Ausführungsform ist und dass dem Fachmann verschiedenartige Modifikationen ersichtlich sind ohne vom Grundgedanken der vorliegenden Erfindung abzuweichen, wie er aus den folgenden Ansprüchen ersichtlich ist. So ist es beispielsweise möglich die Membran direkt mit dem piezoelektrischen Element zu verbinden. Darüber hinaus sind auch andere Formen als die kreisrunden bzw. kreisringförmigen konzentrisch zueinander angeordneten Elemente denkbar. Auch können andere Materialien für Membran und Träger als das erwähnte Edelstahl zum Einsatz kommen.

Dementsprechend müssen jeweils geeignete Schutzgase verwendet werden. Gleichermaßen können auch andere Aktuatoren als piezoelektrische Aktuatoren zum Einsatz kommen, wie zum Beispiel Formgedächtnislegierungen, Schwingkolben, Pumpmotoren, Pumpenkolben, Piezomotoren, Elektromagneten mit Schwingkern, Relais oder dergleichen.

## Patentansprüche

1. Membranvernebler zur Aerosolerzeugung in einer Aerosoltherapievorrichtung, umfassend:
eine Membran (1) mit einem effektiven Bereich (6), in dem mehrere Duchgangslöchern zur Verneblung eines Fluids angeordnet sind, und einem den effektiven Bereich (6) vollumfänglich umgebenden Befestigungsbereich; und
einen flächigen Träger (2) mit einer Öffnung (8), wobei die Membran (1) in der Öffnung (8) angeordnet derart an dem Träger (2) befestigt ist, dass auf einer ersten Seite (9) des Trägers (2) die Vernebelung stattfindet und auf der entgegengesetzten zweiten Seite (10) des Trägers (2) das Fluid an der Membran (1) ansteht, **dadurch gekennzeichnet, dass**
die Membran (1) gewölbt ist und der Befestigungsbereich als auf der ersten Seite (9) des Trägers (2) flächig aufliegender Kragen (7) ausgestaltet ist und vollumfänglich mit dem Träger (2) durch ein Widerstandsschweißverfahren verschweißt ist, und ein Piezoelement (3) an der ersten Seite (9) des Trägers (2) befestigt ist, wobei über eine erste Elektrode (4) und über den Träger (2) eine Wechselspannung angelegt werden kann.

2. Membranvernebler nach Anspruch 1, bei dem die Membran (1) durch ein Mittelfrequenz- oder Kondensatorentladungsschweißverfahren mit dem Träger (2) verschweißt ist.

3. Membranvernebler nach Anspruch 1 oder 2, bei dem der auf dem Träger (2) flächig aufliegende Kragen (7) eine Fläche von weniger als 96 mm² und bevorzugt weniger als 80 mm², mehr bevorzugt weniger als 40 mm² und am meisten bevorzugt weniger als 20 mm² aufweist.

4. Aerosoltherapievorrichtung mit einem Membranvernebler nach einem der Ansprüche 1 bis 3.

5. Verfahren zum Verbinden einer Membran (1) mit einem flächigen Träger (2) bei der Herstellung eines Membranverneblers einer Aerosoltherapievorrichtung, wobei die Membran (1) einen effektiven Bereich (6), in dem mehrere Duchgangslöcher zur Verneblung eines Fluids angeordnet sind, und einen den effektiven Bereiche (6) vollumfänglich umgebenden Kragen (7) zur Verbindung mit dem Träger (2) aufweist, umfassend die Schritte:
flächiges Auflegen des Kragens (7) der Membran (1), der mit dem Träger (2) zu verschweißen ist, auf den Träger (2);
flächiges Anpressen einer im Querschnitt geschlossenen Schweißelektrode auf den Kragen (7); und
Widerstandsschweißen im Bereich des Kragens (7) zum Verbinden von Membran (1) und Träger (2).

6. Verfahren nach Anspruch 5, bei dem der Schritt Widerstandsschweißen, insbesondere Mittelfrequenz- oder Kondensatorentladungsschweißverfahren unter Schutzgasatmosphäre stattfindet.

7. Verfahren nach Anspruch 5 oder 6, bei dem der Verbund aus Membran (1) und Träger (2) und die Schweißelektrode nach dem Schritt Widerstandsschweißen relativ zu einander um einen Winkel weniger als 360° oder deren Kreisvielfaches (+/-360°) gedreht und der Schritt Widerstandsschweißen in dem Schweißbereich nochmals durchgeführt wird, wobei dieser Vorgang bevorzugt wenigstens einmal wiederholt wird.

8. Verfahren nach einem der Ansprüche 5 bis 7, bei dem der Kragen (7) der Membran (1) flächig auf einer Seite (9) des Trägers (2), auf der die Vernebelung stattfindet, auf den Träger (2) aufgelegt wird.

9. Verfahren nach einem der Ansprüche 5 bis 8, bei dem der auf dem Träger (2) flächig aufliegende Kragen (7) eine Fläche von weniger als 96 mm² und bevorzugt weniger als 80 mm², mehr bevorzugt weniger als 40 mm² und am meisten bevorzugt weniger als 20 mm² aufweist.

10. Verfahren nach einem der Ansprüche 5 bis 9, ferner umfassend den Schritt Messen der Ströme und Widerstände während des Widerstandsschweißens zur direkten Qualitätskontrolle, insbesondere Dichtigkeitsmessung.

## Claims

1. Membrane nebuliser for aerosol generation in an aerosol treatment device, comprising:
a membrane (1) having an effective region (6) in which are arranged several through-holes for the nebulisation of a fluid, and a fastening region which surrounds the effective region (6) over the whole circumference; and
a two-dimensional carrier (2) having an opening (8), the membrane (1) arranged in the opening (8) being fastened to the carrier (2) in such a way that nebulisation takes place on a first side (9) of the carrier (2) and the fluid is in contact with the membrane (1) on the opposite, second side (10) of the carrier (2),
**characterised in that** the membrane (1) is curved and the fastening region is designed as a collar (7) which rests flat on the first side (9) of the carrier (2) and is welded to the carrier (2) over the whole circumference by a resistance welding method, and a piezoelement (3) is attached to the first side (9) of the carrier (2), wherein an a.c. voltage can be applied via a first electrode (4) and via the carrier (2).

2. Membrane nebuliser according to claim 1, in which the membrane (1) is welded to the carrier (2) by a medium-frequency or capacitor discharge welding method.

3. Membrane nebuliser according to claim 1 or 2, in which the collar (7) which rests flat on the carrier (2) has a surface area of less than 96 mm² and preferably less than 80 mm², more preferably less than 40 mm² and most preferably less than 20 mm².

4. Aerosol treatment device having a membrane nebuliser according to any of claims 1 to 3.

5. Method for connecting a membrane (1) to a two-dimensional carrier (2) in the manufacture of a membrane nebuliser of an aerosol treatment device, wherein the membrane (1) has an effective region (6) in which are arranged several through-holes for the nebulisation of a fluid, and a collar (7) surrounding the effective region (6) over the whole circumference for connection to the carrier (2), comprising the steps of:
laying the collar (7) of the membrane (1), which is to be welded to the carrier (2), flat on the carrier (2);
pressing a welding electrode of closed cross-section flat against the collar (7); and
resistance welding in the region of the collar (7) for connecting membrane (1) and carrier (2).

6. Method according to claim 5, in which the step of resistance welding, in particular medium-frequency or capacitor discharge welding method, takes place in an inert gas atmosphere.

7. Method according to claim 5 or 6, in which the combination of membrane (1) and carrier (2) and the welding electrode after the step of resistance welding are rotated relative to each other about an angle of less than 360° or a multiple thereof (+/-360°), and the step of resistance welding is performed again in the welding region, this operation preferably being repeated at least once.

8. Method according to any of claims 5 to 7, in which the collar (7) of the membrane (1) is laid flat on the carrier (2) on a side (9) of the carrier (2) on which nebulisation takes place.

9. Method according to any of claims 5 to 8, in which the collar (7) which rests flat on the carrier (2) has a surface area of less than 96 mm² and preferably less than 80 mm², more preferably less than 40 mm² and most preferably less than 20 mm².

10. Method according to any of claims 5 to 9, further comprising the step of measuring the currents and resistances during resistance welding for direct quality control, in particular leak testing.

## Revendications

1. Nébuliseur à membrane destiné à produire un aérosol dans un dispositif d'aérosolthérapie, comprenant :
une membrane (1) avec un champ efficace (6) où sont ménagés plusieurs trous de passage pour la nébulisation d'un fluide, et une zone de fixation entourant toute la périphérie du champ efficace (6) ; et
un support plan (2) avec une ouverture (8), la membrane (1) étant disposée dans l'ouverture (8) contre le support (2) de telle sorte que la nébulisation est réalisée sur une première face (9) du support (2) et que le fluide reste présenté contre la membrane (1) sur la deuxième face (10) opposée du support (2), **caractérisé en ce que**
la membrane (1) est incurvée et la zone de fixation est réalisée comme rebord (7) appliqué à plat sur la première face (9) du support (2) et soudé au support (2) sur toute sa périphérie au moyen d'un procédé de soudage par résistance, et **en ce qu'**un élément piézo-électrique (3) est fixé contre la première face (9) du support (2), une tension alternative pouvant être appliquée sur une première électrode (4) et sur le support (2).

2. Nébuliseur à membrane selon la revendication 1, où la membrane (1) est soudée au support (2) au moyen d'un procédé de soudage moyenne fréquence ou de soudage par décharge de condensateur.

3. Nébuliseur à membrane selon la revendication 1 ou la revendication 2, où le rebord (7) appliqué à plat sur le support (2) a une superficie inférieure à 96 mm², avantageusement inférieure à 80 mm², tout particulièrement inférieure à 40 mm² et préférentiellement inférieure à 20 mm².

4. Dispositif d'aérosolthérapie comprenant un nébuliseur à membrane selon l'une des revendications 1 à 3.

5. Procédé de raccordement d'une membrane (1) à un support plan (2) lors de la fabrication d'un nébuliseur à membrane d'un dispositif d'aérosolthérapie, la membrane (1) présentant un champ efficace (6) où sont ménagés plusieurs trous de passage pour la nébulisation d'un fluide et un rebord (7) entourant toute la périphérie du champ efficace (6) pour le raccordement au support (2), comprenant les étapes de :
application à plat sur le support (2) du rebord (7) de la membrane (1) à souder au support (2) ;
serrage à plat sur le rebord (7) d'une électrode de soudage à section transversale fermée ; et
soudage par résistance au niveau du rebord (7) pour le raccordement de la membrane (1) et du support (2).

6. Procédé selon la revendication 5, où l'étape de soudage par résistance, en particulier de soudage moyenne fréquence ou de soudage par décharge de condensateur, est exécutée sous atmosphère de gaz protecteur.

7. Procédé selon la revendication 5 ou 6, où après l'étape de soudage par résistance, l'assemblage de la membrane (1) et du support (2) et l'électrode de soudage sont tournés suivant un angle inférieur à 360° ou d'un multiple circulaire (+/-360°) l'un par rapport à l'autre, et où l'étape de soudage par résistance est à nouveau exécutée dans la zone de soudage, ce processus étant préférentiellement répété au moins une fois.

8. Procédé selon l'une des revendications 5 à 7, où le rebord (7) de la membrane (1) est appliqué à plat sur le support (2), sur une face (9) du support (2) où se produit la nébulisation.

9. Procédé selon l'une des revendications 5 à 8, où le rebord (7) appliqué à plat sur le support (2) a une superficie inférieure à 96 mm², avantageusement inférieure à 80 mm², tout particulièrement inférieure à 40 mm² et préférentiellement inférieure à 20 mm².

10. Procédé selon l'une des revendications 5 à 9, comprenant en outre l'étape de mesure des courants et des résistances pendant le soudage par résistance pour un contrôle immédiat de qualité, en particulier une mesure d'étanchéité.
